# EUROPEAN PATENT SPECIFICATION

(11) **EP 0 421 725 B1**
(45) Date of publication and mention of the grant of the patent: **15.05.1996**
(21) Application number: 90310766.2
(22) Date of filing: 02.10.1990
(51) Int. Cl.: C12Q 1/68, C07H 21/04

(54) **Nucleic acid probes for the detection of Lyme disease spirochetes**
Nukleinsäuresonden zum Nachweis von Lyme-Krankheit verursachenden Spirocheten
Sondes d'acides nucléiques pour la détection de spirochètes provoquant la maladie de lyme

(30) Priority: 02.10.1989 US 416072
(43) Date of publication of application: 10.04.1991
(73) Proprietor: AMOCO CORPORATION, Chicago Illinois 60680-0703 (US)
(72) Inventor: Weisburg, William G., Milford, MA 01757 (US)
(74) Representative: Sheard, Andrew Gregory

(56) References cited:
- EP-A- 0 272 009
- WO-A-89/05359
- WO-A-90/15157
- CHEMICAL ABSTRACTS, vol. 107, no. 9, 31st August 1987, page 362, abstract no. 73936x, Columbus, Ohio, US; L. HAYES et al.: "A short peptide sequence is conserved between three tick-borne Borrelia",& BIOCHEM. SOC. TRANS. 1987, 15(4), 644/
- CHEMICAL ABSTRACTS, vol. 112, no.9, February 1990, page 410,abstract no. 173093y, Columbus, Ohio, US; P.A. ROSA et al.: "A specific and sensitive assay for the Lyme disease spirochete Borrelia burgdorferi using the polymerase chain reaction", & J. INFECT. DIS. 1989, 160(6), 1018-29/
- CHEMICAL ABSTRACTS, vol. 110, no. 21, May 1989, page 406, abstract no. 189215c, Columbus, Ohio, US; R.B. LeFEBVRE et al.: "Characterization of Borrelia burgdorferi isolates by restriction endonuclease analysis and DNA hybridization",& J. CLIN. MICROBIOL. 1989, 27(4), 636-9/
- CHEMICAL ABSTRACTS, vol. 113, no. 5, 30th July 1990, page 294, abstract no. 37190q, Columbus, Ohio, US; D.C. MALLOY et al.: "Detection of Borrelia burgdorferi using the polymerase chain reaction", & J. CLIN. MICROBIOL. 1990, 28(6), 1089-93/
- BIOLOGICAL ABSTRACTS, vol. 88, no. 7, April 1989, abstract no. 75033, Philadelphia, PA, US; T.G. SCHWAN et al.: "Identification of Borrelia burgdorferi and Borrelia hermsii using DNA hybridization probes", & J. CLIN. MICROBIOL. 27(8): 1734-1738. 1989/
- J. CLIN. CHEM. BIOCHEM., vol.25, no.9, 1987, page 573; U.B. GöBEL et al.: "Synthetic oligodeoxynucleotides complementary to ribosomal RNA as probes for rapid detection and identification of fastidious pathogenic bacteria"/

## Description

This invention relates to detection of bacteria belonging to the species Borreliaburgdorferi and related species of tick-borne spirochetes capable of causing human and veterinary disease. More specifically, it provides nucleic acid probes and compositions along with methods for their use for the specific detection of Lyme disease causing bacteria.

Lyme borreliosis, Lyme disease, Lyme arthritis, Bannwarth's syndrome, or erythema chronicum migrans (ECM) are synonymous designations for a zoonotic spirochetal infection transmitted by the bite of ticks of the genus Ixodes. Although the disease was known in Europe for quite some time, it was not discovered in the United States until a 1975 arthritic epidemic occurred in Connecticut. A presumptive etiological agent was isolated from a tick in 1982 (Burgdorfer et.al, Science 216:1317-1319, 1982). In 1984, the spirochete was shown to be a member of the genus Borrelia, and formally named Borreliaburgdorferi (Johnson et.al, Int. J. Syst. Bacteriol. 34:496-497, 1984). As such, it is an evolutionary relative of Borrelia hermsii, Borrelia turicatae, Borrelia anserina, and other members of this arthropod associated genus of spiral bacteria.

Lyme disease is a serious chronic borrelial infection characterized by a diversity of symptoms at various stages. Approximately 3 to 14 days following the initiating tick bite, symptoms may include fever, flu-like illness, and the appearance of the ECM skin rash. Stage two, occurring weeks to months after the initial bite,includes further skin involvement, arthritis, nervous system complaints, and cardiac pathology. Stage three is characterized by more severe arthritis and nervous system complications.

Diagnosis of Lyme disease is generally either differential or dependent on host antibody response. Isolation and culture of these bacteria as a diagnostic method is not considered technically or economically feasible. Differential diagnosis relies on time of year (tick season), residence in an endemic area, recollection of tick-bite history, and ECM rash. This diagnostic scheme suffers, respectively, from the chronic nature of the disease, infection of travellers, the incredibly small size of the ticks, and that not all patients experience the ECM rash. Diagnosis based on antibody response requires the seroconversion of infected individuals toward production of anti-B. burgdorferi antibodies. While the antibody approach works for many diagnostic problems, it fails for Lyme diagnosis for the following reasons:
- The combination of low numbers of spirochetes and antigenically bland outermembrane yields weak host immune response.
- Seroconversion takes some period of time after infection. During the height of spirochetemia, within weeks of the initial tick bite, a patient presenting with fever and ECM may fail to evince antibody.
- The antibody response is transitory. During Stage two or Stage three, when circulating bacteria are fewest, antibody titers may be very low.
- Cell mediated immunity may detract from circulating antibody signal, that is, classical host IgC and IgM response is decreased or absent due to masked antigens.
- Most of the antibody tests rely on whole Borrelia burgdorferi preparations as target antigens. The major problem with this approach is cross-reactivity with an immunological response to other bacterial challenges. Most noteworthy is cross-reaction with anti-Treponema pallidum (syphilis) antibodies.

It is an aspect of the present invention to provide non-immunologically based assays thereby avoiding associated problems and to provide nucleic acid probes which are specific for tick-borne spirochetes capable of causing Lyme disease and related morbidity.

It is another aspect of the present invention to provide probes and assays particularly specific for the type strain of the species, Borrelia burgdorferi B31, American Type Culture Collection strain number 35210.

It is yet another aspect of the present invention to provide nucleic acid probes which can hybridize to target regions which can be rendered accessible to probes under normal assay conditions.

While Kohne et al. (Biophysical Journal 8:1104-1118, 1968) discuss one method for preparing probes to rRNA sequences, they do not provide the teaching necessary to make Borrelia burgdorferi specific probes or, in fact, any other probes to detect spirochete bacteria.

EP-A-0272009 describes a method for preparing nucleic acid probes complementary to the small rRNA of a target non-viral organism and to permit specific identification thereof. There is, however, no disclosure of the probes of the present invention. The Abstract by Göbel and Chuba J. *Clin. Biochem* **25** (9) page 573 (1987) describes how *B. burgdorferi* could be identified in a sample using oligonucleotide probes targeted to specific rRNA sequences. It does not, however, disclose which sequences would be of use in such an assay.

WO-A-9015157 is included in the description of the prior art as a document falling within the terms of Article 54(3) of the European Patent Convention and it discloses the partial sequence of the probe/primers 1637 and 1643. However, there is no disclosure of the probes 1616, 1617, 1618, 1619, 1620, 1621 or 1622 of the present invention.

Pace and Campbell (Journal of Bacteriology 107:543-547, 1971) discuss the homology of ribosomal ribonucleic acids from diverse bacterial species and a hybridization method for quantitating such homology levels. Similarly, Sogin, Sogin and Woese (Journal of Molecular Evolution 1:173-184, 1972) discuss the theoretical and practical aspects of using primary structural characterization of different ribosomal RNA molecules for evaluating phylogenetic relationships. Fox, Pechman and Woese (International Journal of Systematic Bacteriology 27:44-57, 1977) discuss the comparative cataloging of 16S ribosomal RNAs as an approach to prokaryotic systematics. These references, however, fail to relieve the deficiency of Kohne's teaching vith respect to Lyme spirochetes and in particular, do not provide Lyme spirochete specific probes useful in assays for detecting Lyme disease or its etiological agent, Borrelia burgdorferi and relatives. Ribosomes are of profound importance to all organisms because they serve as the only known means of translating genetic information into cellular proteins, the main structural and catalytic elements of life. A clear manifestation of this importance is the observation that all cells have ribosomes.

Ribosomes contain three distinct RNA molecules which, at least in Escherichia coli, are referred to as 55, 16S and 23S rRNAs. These names historically are related to the size of the RNA molecules, as determined by their sedimentation rate. In actuality, however, ribosomal RNA molecules vary substantially in size between organisms. Nonetheless, 5S, 16S, and 23S rRNA are commonly used as generic names for the homologous RNA molecules in any bacteria, and this convention will be continued herein. An additional convention used herein designates sequence position numbers analogous to those of the Escherichia coli 16S rRNA sequence (Brosius et al., PNAS (USA) 75:4801-4805, 1978).

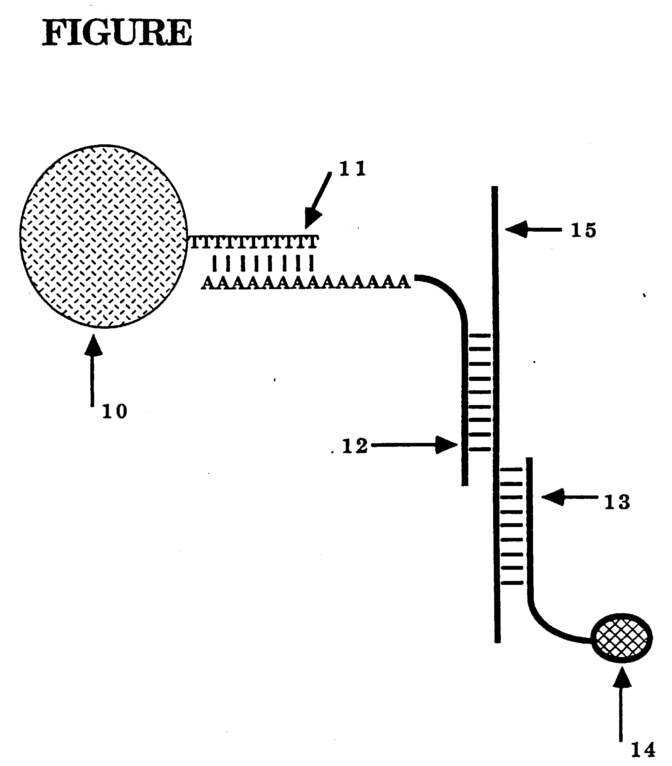

As used herein, probe(s) refer to synthetic or biologically produced nucleic acids (DNA or RNA) which, by design or selection, contain specific nucleotide sequences that allow them to hybridize under defined predetermined stringencies, specifically (i.e., preferentially, see next paragraph) to target nucleic acid sequences. In addition to their hybridization properties, probes also may contain certain constituents that pertain to their proper or optimal functioning under particular assay conditions. For example, probes may be modified to improve their resistance to nuclease degradation (e.g. by end capping), to carry detection ligands (e.g. fluorescien, 32-P, biotin, etc.), or to facilitate their capture onto a solid support (e.g., polydeoxyadenosine "tails"--see Figure 2). Such modifications are elaborations on the basic probe function which is its ability to usefully discriminate between target and non-target organisms in a hybridization assay.

Hybridization traditionally is understood as the process by which, under predetermined reaction conditions, two partially or completely complementary strands of nucleic acid are allowed to come together in an antiparallel fashion (one oriented 5′ to 3′, the other 3′ to 5′) to form a double-stranded nucleic acid with specific and stable hydrogen bonds, following explicit rules pertaining to which nucleic acid bases may pair with one another. The high specificity of probes relies on the low statistical probability of unique sequences occurring at random as dictated by the multiplicative product of their individual probabilities. These concepts are well understood by those skilled in the art.

The stringency of a particular set of hybridization conditions is defined by the base composition of the probe/target duplex, as well as by the level and geometry of mispairing between the two nucleic acids.

Stringency may also be governed by such reaction parameters as the concentration and type of ionic species present in the hybridization solution, the types and concentrations of denaturing agents present, and/or the temperature of hybridization. Generally, as hybridization conditions become more stringent, longer probes are preferred if stable hybrids are to be formed. As a corollary, the stringency of the conditions under which a hybridization is to take place (e.g., based on the type of assay to be performed) will dictate certain characteristics of the preferred probes to be employed. Such relationships are well understood and can be readily manipulated by those skilled in the art.

As a general matter, dependent upon probe length, such persons understand stringent conditions to mean approximately 35°C-65°C in a salt solution of approximately 0.9 molar.

According to the first aspect of the present invention there is provided a nucleic acid fragment capable of hybridising, under predetermined stringency conditions, to ribosomal RNA (rRNA) or rDNA genes (rDNA) of Borrelia burgdorferi. The fragment is complementary to at least 90% of a sequence comprising any ten consecutive nucleotides within the nucleotide sequences of one of the following probes: Probe 1616, Probe 1617, Probe 1618, Probe 1619, Probe 1620, Probe 1621 or Probe 1622; whose sequences are defined below.

According to the second aspect of the present invention there is provided a nucleic acid fragment capable of hybridising, under predetermined stringency conditions, to rRNA or rDNA genes (rDNA) of Borrelia burgdorferi. The fragment is homologous to at least 90% of a sequence comprising any ten consecutive nucleotides within probe 1616, probe 1617, probe 1618, probe 1619, probe 1620, probe 1621 or probe 1622.

Nucleic acid sequences in accordance with either the first or second aspect of the present invention may comprise probe 1616, probe 1617, probe 1618, probe 1619, probe 1620, probe 1621 or probe 1622 or one of their complementary sequences. The present invention also includes a set of probes comprising at least two nucleic acid fragments in accordance with either the first or the second aspect of the present invention, or a nucleic acid sequence complementary thereto. The set of probes may also comprise a first nucleic acid fragment as described previously and a second nucleic acid that is probe/primer 1643 or probe/primer 1637 which have a nucleotide sequence as defined below.

According to a third aspect of the present invention a method is provided for detecting the etiological agent of Lyme disease or borreliosis in a sample. The method comprises the steps of:
(a) contacting the sample with at least one nucleic acid fragment in accordance with either the first or second aspects of the present invention or a nucleic acid sequence complementary thereto; under conditions that allow the fragment to hybridise to rRNA or rDNA of a Borrelia species causing Lyme disease, if present in the sample, whereby hybrid nucleic acid complexes are formed; and
(b) detecting the hybrid nucleic acid complexes as an indication of the presence of a Borrelia species.

According to a fourth aspect of the present invention there is provided a method for detecting the etiological agent of Lyme disease or borreliosis in a sample, comprising the steps of:
(a) contacting the sample with a nucleic acid fragment comprising probe/primer 1637 or probe/primer 1643; under conditions that allow the fragment to hybridise to rRNA or rDNA of a Borrelia species causing Lyme disease if present in the sample, whereby hybrid nucleic acid complexes are formed;
(b) detecting the hybrid nucleic acid complexes by additionally contacting the sample with a second nucleic acid fragment comprising probe 1616, probe 1617, probe 1618, probe 1619, probe 1620, probe 1621 or probe 1622 or one of their complementary sequences; and
(c) amplifying the 16S rRNA gene sequence of the Borrelia species, if present, by polymerase chain reaction.

Therefore the probes of the present invention provide the basis for development of a valuable nucleic acid hybridization assay for the specific detection of Lyme disease, or its etiological agent, in clinical samples of blood, urine, cerebrospinal fluid, skin biopsy, saliva, synovial fluid, or other tissue or fluid samples from human patients or veterinary subjects. The probes also provide the basis for testing the tick vectors of Lyme disease--the genus Ixodes--to assess infectivity rates or endemic range.

In our experience nucleic acid hybridization based assays have been discovered to impart enhanced performance capabilities with respect to most currently available, microbiological or immunological methods for detection of bacteria in test samples, generally including:
a) increased sensitivity; i.e., the ability to detect said bacteria in a given sample more frequently;
b) potentially significant reductions in assay cost due to the use of inexpensive reagents and reduced labor;
c) accurate identification of even biochemically unusual strains of the target bacteria, or bacteria with dramatically different outer membrane proteins;
d) direct assay for the presence of the bacterium and consequent potential to quantify the etiological agents;
e) assay independent of the host's immune response schedule--much earlier detection is possible;
f) direct testing allows the monitoring of the efficacy of an antibiotic regime;
g) potential to detect said etiological agent in samples of tissue normally low in antibody titer such as skin.

It has been discovered that other advantages incurred by directing the probes of the present invention against rRNA include the fact that the rRNAs detected constitute a significant component of cellular mass. Although estimates of cellular ribosome content vary, actively growing Borrelia burgdorferi may contain upwards of 20,000 ribosomes per cell, and therefore 20,000 copies of each of the rRNAs (present in a 1:1:1 stiochiometry in ribosomes). In contrast, other potential cellular target molecules such as genes or RNA transcripts thereof, are less ideal since they are present in much lower abundance. A further unexpected advantage is that the rRNAs (and the genes specifying them) appear not to be subject to lateral transfer between contemporary organisms. Thus, the rRNA primary structure provides an organism-specific molecular target, rather than a gene-specific target as would likely be the case, for example of a plasmid-borne gene or product thereof which may be subject to lateral transmission between contemporary organisms.

The discovery that probes could be generated with the extraordinary inclusivity and exclusivity characteristics of those of the present invention with respect to the detection of the etiological agent of Lyme disease, Borrelia burgdorferi and its close relatives was unpredictable and unexpected.

Further understanding of the principles and aspects of the present invention may be made by reference to the table wherein:

Table 1: Exemplifies the inclusivity and exclusivity behavior of the preferred probes toward a representative sampling of Borreliaburgdorferi strains from the United States and Europe, including both clinical and tick isolates, in a dot blot hybridization assay; and by reference to the figure showing schematic representation of a dual probe capture/detector assay.

### Probe Development Strategy

The first step taken in the development of the probes of the present invention involved identification of regions of 16S rRNA which potentially could serve as target sites for Lyme spirochete specific nucleic acid probes. As a practical matter, it is difficult to predict, a priori, which non-Borrelia organisms might be present in any test sample. Of particular import is that probe combinations do not inadvertently detect as Lyme-positive samples actually containing the syphilis spirochete, Treponema pallidum.

Because of the large number of such potential non-Lyme agent bacteria, demonstrating exclusivity for any given probe sequence is not only unpredictable but also extremely difficult and laborious. A more rigorous criterion was adopted to obviate the need to know what non-Borrelia bacteria might be present in all test samples that ultimately will be screened using the probes.

This entailed knowledge of the phylogenetic relationships among spirochetes and between spirochetes and other groups of bacteria.

Specifically, an operating but previously unproven hypothesis was adopted that the exclusivity criterion could be satisfied by determining that if a particular target region in Borrelia burgdorferi rRNA could be identified which was sufficiently different from the homologous region in the rRNA of representative yet close evolutionary relatives of Borrelia burgdorferi, then a probe to such a sequence could be used to distinguish between Borrelia burgdorferi and the relatives by hybridization assay. Based on phylogenetic observations, it then was extrapolated that rRNA sequences of more distantly related organisms, even though their actual identity may not necessarily be known, should be predictably different in a particular region of sequence than the aforementioned close evolutionary relative of Borrelia burgdorferi. However, it cannot be predicted, a priori, whether such regions exist or if they do, where within the rRNA such regions will be located.

Determination of the 16S rRNA sequence from the type strain of Borrelia burgdorferi, by standard laboratory methods, and subsequent comparison to sequences of other spirochete 16S rRNAs and to sequences from other blood infectious organisms narrows considerably the search for worthwhile target sequences within the 16S rRNA. Additional evaluation of 16S rRNA sequences from tick-borne bacterial genera, such as Rickettsia and Ehrlichia, capable of causing significant human morbidity or mortality, also contributed to ultimate probe design.

Probes may be synthesized by any suitable chemical or biological method. In particular, DNA probes may be synthesized by automated phosphoramide chemistry using cyanoethyl phosphoramides (Beaucage and Carruthers Tetrahedron Letters 24,245 (1981)). RNA probes can be made by transcription of the corresponding DNA sequences.

### Physical Description of the Probes

The probe selection strategy yielded a number of probes useful for identifying Lyme spirochetes in samples. The following preferred oligonucleotide probes are disclosed herein:

PROBE 1616: 5'-GCCACTGAATGTATTGCTACATCCCGTTG-3' SEQ ID. 1

PROBE 1617: 5'-CATCAATTAACAAATTAACTGACCTTATT-3' SEQ ID. 2

PROBE 1618: 5'-CCTCATTTATAAAAGAATTTTACAATCTTTCGACC-3' SEQ ID. 3

PROBE 1619: 5'-CATCACTTTGTCATTTCCTACAAAGCTTA-3' SEQ ID. 4

PROBE 1620: 5'-GTTAGCTTCGGTACTAACTTTTAGTTAACACC-3' SEQ ID 5

PROBE 1621: 5'-CTTATCTGAGTCCCCACCATTACATGCTGGTAACAG-3' SEQ ID. 6

PROBE 1622: 5'-CATACCTTAAATACCTTCCTCCCTTACGGGTTAGAATAATA SEQ ID. 7
GCTTCGGGTATCCTCAACTCGGG-3'

These probes target regions within the 16S rRNA with the following analogous target position numbers of Escherichia coli 16S rRNA (Brosius et al., PNAS (USA) 75:4801-4805, 1978) and designated target sequences:

PROBE 1616 TARGETS REGION 63-106: 5'-CAAACGGGAUGUAGCAAUACAUUCAGUGGC-3'
SEQ ID. 8
PROBE 1617 TARGETS REGION 178-194: 5'-AAUAAGGUCAGUUAAUUUGUUAAUUGAUG-3'
SEQ ID. 9
PROBE 1618 TARGETS REGION 416-450: 5'-GGUCGAAAGAUUGUAAAAUUCUUUUAUAAA
UGAGG-3' SEQ ID. 10
PROBE 1619 TARGETS REGION 453-481: 5'-UAAGCUUUGUAGGAAAUGACAAAGUGAUG-3'
SEQ ID. 11
PROBE 1620 TARGETS REGION 829-866: 5'-GGUGUUAACUAAAAGUUAGUACCGAAGCU
AAC-3' SEQ ID. 12
PROBE 1621 TARGETS REGION 1122-1156: 5'-CUGUUACCAGCAUGUAAUGGUGGG
GACUCAGAUAAG-3' SEQ ID. 13

PROBE 1622 TARGETS REGION 1414-1475: 5'-CCCGAGUUGAGGAUACCCGAAGCUAUUAUU
CUAACCCGUAAGGGAGGAAGGUAUUUAAGGU
AUG-3' SEQ ID. 14

The identification of probe target regions is one of the key aspects of this invention, and enabled the identification of the preferred probes. Each of the probe target regions contains a fundamental core of key nucleotides crucial for creating mismatches with potentially cross-reactive, non-Borrelia organisms. The redesign of the probes by way of shortening the ends, or shifting the probe slightly to one side of the probe target would not be considered a novel redesign of the probe and is contemplated as an equivalent of the probes designated herein.

Two additional oligonucleotides discussed in this invention include:

PROBE/PRIMER 1643: 5'-CCGAATTCGTCGACAACAGAGTTTGATCCTGGCTTAG-3'
SE[?] ID. 15
PROBE/PRIMER 1637: 5'-CCCGGGATCCAAGCTTAAGGAGGTGATCCAGCC-3'
SEO ID. 16

Probe/Primer 1643 is designed to hybridize to the 165 rDNA gene strand complementary to the Borrelia 16S rRNA at its 5' end consisting of the following sequence:

PROBE/PRIMER 1643 TARGET: 5'-CTAAGCCAGGATCAAACTCT-3' SEQ ID. 17

Probe/Primer 1637 is designed to hybridize to the opposite DNA strand of the preceding target at the 16S rRNA 3' end:

PROBE/PRIMER 1637 TARGET:  5'-GGCTGGATCACCTCCTT-3'    SE[?] ID. 18

Oligonucleotides 1643 and 1637 are designed for use in assays employing amplification, by the polymerase chain reaction method, of almost the entire 165 rRNA gene (rDNA) of Borrelia burgdorferi and relatives. They are more fully discussed in commonly assigned copending USSN 359,158 entitled Universal Eubacteria Nucleic Acid Probes and Methods, by Lane et al. equivalent to EP-A-0 431 149 and are used in Example 6 ragarding Borrelia-specific amplification and detection.

### Examples

### Example 1: Dot-Blot Analysis of Probe Hybridization Behavior

Dot-blot analysis, in accordance with well known procedures, involves immobilizing a nucleic acid or a population of nucleic acids on a filter such as nitrocellulose, nylon, or other derivatized membranes which can readily be obtained commercially, specifically for this purpose. Either DNA or RNA can be easily immobilized on such a filter and subsequently can be probed or tested for hybridization under any of a variety of conditions (i.e., stringencies) with nucleotide sequences or probes of interest. Under stringent conditions, probes whose nucleotide sequences have greater complementarity to the target will exhibit a higher level of hybridization than probes containing less complementarity.

Probes 1616, 1617, 1618, 1619, 1620, 1621, and 1622 were tested in a dot-blot format. One hundred nanograms of RNA, purified by phenol extraction and centrifugation through cesium triflouracetate gradients, was denatured and spotted on a nylon membrane. Probes were isotopically labelled with the addition of a 32-Phosphorous moiety to the 5' end of the oligonucleotide. Hybridization of probes occurred, at temperatures indicated in Table 1, in the presence of 1.08 M sodium chloride, 60 mM sodium phosphate, and 6 mM ethylenediamine tetraacetic acid, pH 7.4. Unhybridized probe was removed by washing at a salt concentration one-third of the hybridization condition. The filters were exposed to X-ray film and the intensity of hybridization signals was evaluated after three hours of exposure. In the table, "+" represents strong hybridization, "+-" represents a faint signal, and "-" designates no signal from hybridization.

The dot blot results shown in Table 1 indicate heterogeneity among the Borrelia burgdorferi strains and a close relationship to the other Borrelia species.

### Example 2: Dual Probe Hybridization

In actual practice, many applications of these probes vould employ a pair of probes being used simultaneously in a "sandwich" hybridization scheme of "capture" probe and "detector" probe as shown in Figure 1. The capture probe¹ ideally would be a bifunctional polynucleotide manufactured by adding a homopolymeric 3' poly-A tail to a probe with high target specificity. The tail would, in turn, hybridize to the complementary homopolymer¹¹ on a solid surface¹⁰, such as a glass bead, a filter disc, or a magnetic particle. Hybridization of the capture probe¹ to its target¹⁵, in this case Lyme spirochete 16S rRNA, would complex the target¹⁵ with the solid support¹⁰. The detector probe¹³, advantageously also with some degree of specificity, would be part of a preferred detection scheme relying on radioactivity, fluorescence, chemiluminescence, color, etc. (detection moiety¹⁴) which would report the presence of the entire hybridization complex.

For specific detection of the U.S. Lyme etiological agent, Borrelia burgdorferi, for example, a combination of probe 1617 for capture and, 1616, 1618, 1619, 1620, 1621, or 1622 for detection would be the most preferred pair of probes in the preferred assay system. Probe 1620, as a capture probe, for example, would be more completely inclusive for all of the Borrelia burgdorferi, but under certain hybridization conditions is inclusive for two other American tick-borne zoonotic pathogens, Borrelia hermsii and Borrelia turicatae, as well.

### Example 3: Clinical diagnosis of Lyme disease from human blood sample

Blood is preferably processed so as to yield total nucleic acid content, such as by sonication, vortexing vith glass beads, detergent lysis using an agent such as SDS or chemical treatment, or alternatively, bacterial cells are partially purified by, for example, the DuPont Isolator System, followed by cell lysis. Probe 1620, with a polymeric stretch of Adenosine residues attached, and Probe 1621 including a 32-Phosphorous tag, are then ideally combined with the sample in a chaotropic buffer such as guanidinium isothiocyanate. Magnetic particle beads which have been derivatized with oligo-Thymidine also are advantageously included. A hybridization complex comprising the magnetic bead, Probe 1620, target 16S rRNA of Borrelia burgdorferi or one of its close relatives and Probe 1621 forms. The exterior presence of a magnet near the bottom of the reaction tube will cause the magnetic particle-hybridization complex to adhere to the interior side of the tube thereby enabling removal of the unreacted components such as the sample matrix, unbound probe, etc. Repeated rehydration and denaturation of the bead-probe-target complex is advantageously performed to enable significant background reduction (as more fully described in USSN Collins 922,155 equivalent to EP-A-0 265 244). In this example, final detection could entail spotting the beads on membrane and assaying by autoradiography.

### Example 4: Clinical diagnosis of Lyme disease from other samples.

The procedures set forth in Example Three are ideally followed except, however, that in substitution for the blood sample, one may use a urine sample, skin sample, human synovial fluid, biopsy, or other tissue or fluid. Obvious minor modifications may have to be made in order to accomodate and process the alternative samples to thereby adequately present the target nucleic acid suitably.

### Example 5: Veterinary diagnosis of Lyme disease

The procedures set forth in Example Three is ideally followed except, however, the sample to be tested is a sample from dog, cat, horse, or other animal. Such a sample may be of the type described in Examples 3 or 4.

### Example 6: Clinical diagnosis of Lyme disease from human sample employing polymerase chain reaction amplification of Borrelia rDNA

Sample processing is designed so as to yield DNA such as the procedures described in Example 3. Probe/Primer 1643 and Probe/Primer 1637 are employed in conjunction with the clinical sample in a polymerase chain reaction. The resultant material can then advantageously be assayed in a "sandwich" hybridization (Example 2) with any of the probes set forth herein. The polymerase chain reaction can, itself, ideally be made highly specific by employing Probe 1643 in conjunction with, for example, Probe 1620 (see Figure 1). Detection could subsequently be accomplished using any of the Probes: 1616, 1617, 1618, or 1619, or could be measured by incorporation of nucleotide triphosphates into polynucleotides of approximately 850 base pairs in length.

### Example 7: Test of individual ticks for Borrelia spirochete infection

In order to ascertain the infective potential of ticks within a given area known to be endemic, ticks immobilized by etherization or by freezing are squashed onto nitrocellulose or nylon membrane. Many ticks thusly prepared may be processed simultaneously on the same filter; each individual tick should be approximately 1 cm from any other sample. Filters should then be denatured in base and neutralized as recommended by the manufacturer. Probe 1622, or alternatively, one of the other probes disclosed herein, labelled to high specific activity with 32-Phosphorous is hybridized to the tick panel. After removal of unhybridized probe by washing, the filter is exposed to X-ray film for from 1 to 10 days. Dark spots on the film indicate ticks which contain Lyme spirochetes.

### Example 8: Test of individual ticks for Borrelia spirochete infection by liquid hybridization

A liquid hybridization assay has the advantage of higher sensitivity and ability to perform more than one assay on the same sample, and is therefor preferred over the procedures of Example 7. In this example, individual ticks, such as those removed from human skin are homogenized in chaotropic buffer, and a fraction of this homogenate is assayed in a format analogous to the "sandwich" method described in Example 2.

### Example 9: Test of ticks from a locality for endemic presence of Lyme disease in an area

A number of ticks from an individual collection site are pooled and homogenized in chaotropic buffer, and assayed as provided in Examples 7 or 8. Positive signal indicates presence of the spirochete in the area, but makes no quantitative evaluation of prevalence.

### Example 10: In situ hybridization of probe to skin biopsy sample

Skin is one of the better sites to visualize spirochetes, particularly during stage one Lyme disease. A probe, such as for example Probe 1622, is labelled with rhodamine or fluorescein. Skin biopsy, on a slide, is incubated with the fluorescent probe under conditions which foster hybridization and then washed free of unbound probe. Observed under fluorescence microscopy, flourescence of characteristically spiral bacterial cells would be visible.

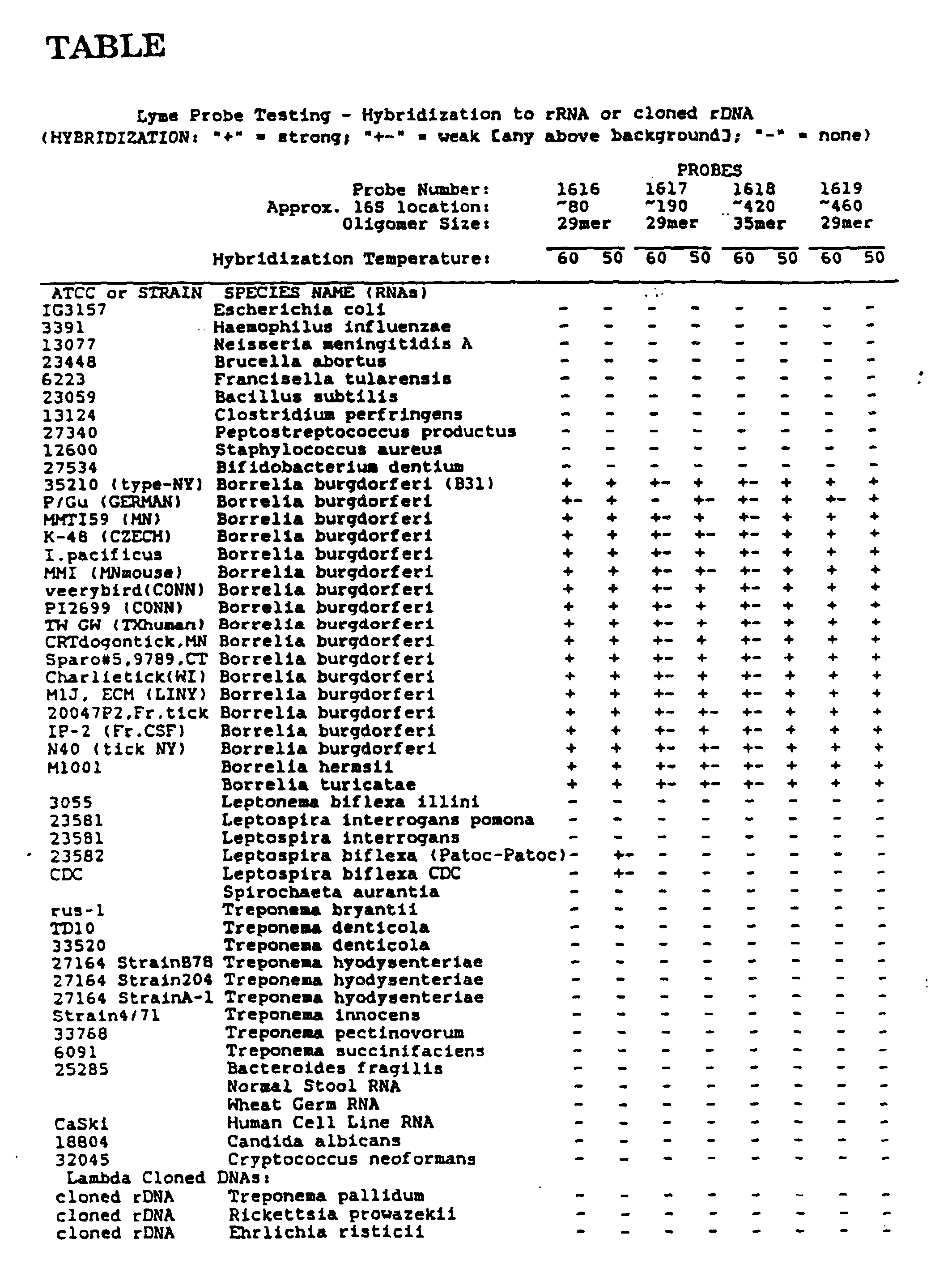

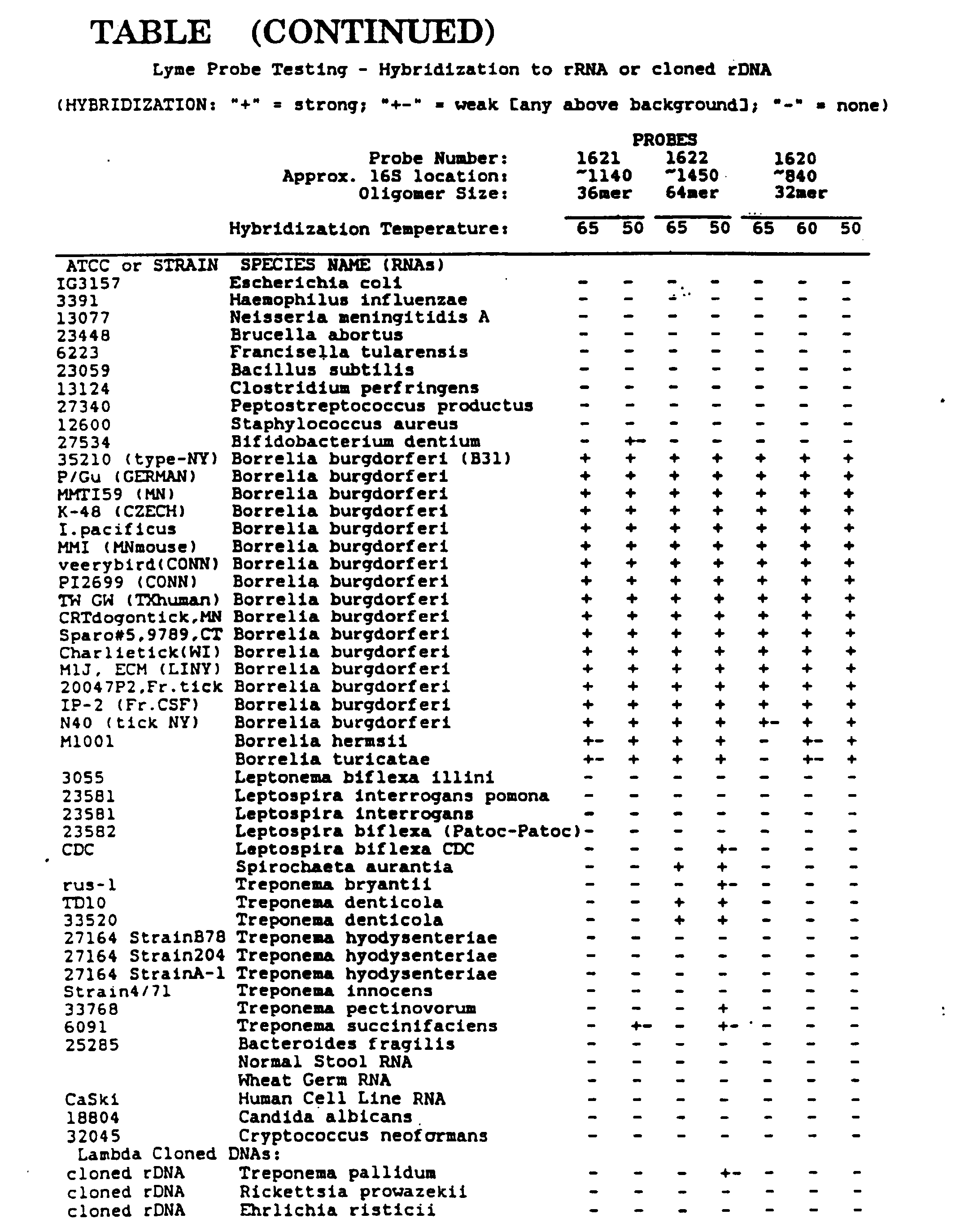

### SEQUENCE LISTING

SEQ. ID NO: 1
SEQUENCE LENGTH: 29 bases
SEQUENCE TYPE: Nucleotide
STRANDEDNESS: single
TOPOLOGY: Linear
MOLECULAR TYPE: synthetic or biological DNA or RNA
HYPOTHETICAL: No
SENSE OR ANTI-SENSE: Sense
FEATURES: Referred to in the specification as probe 1616
PROPERTIES: Probe for selective detection of Borrelia burgdorferi
SEQUENCE:

GCCACTGAAT GTATTGCTAC ATCCCGTTG

### SEQUENCE LISTING

SEQ. ID NO: 2
SEQUENCE LENGTH: 29 bases
SEQUENCE TYPE: Nucleotide
STRANDEDNESS: single
TOPOLOGY: Linear
MOLECULAR TYPE: synthetic or biological DNA or RNA
HYPOTHETICAL: No
SENSE OR ANTI-SENSE: Sense
FEATURES: Referred to in the specification as probe 1617
PROPERTIES: Probe for selective detection of Borrelia burgdorferi
SEQUENCE:

CATCAATTAA  CAAATTAACT  GACCTTATT

### SEQUENCE LISTING

SEQ. ID NO: 3
SEQUENCE LENGTH: 35 bases
SEQUENCE TYPE: Nucleotide
STRANDEDNESS: single
TOPOLOGY: Linear
MOLECULAR TYPE: synthetic or biological DNA or RNA
HYPOTHETICAL: No
SENSE OR ANTI-SENSE: Sense
FEATURES: Referred to in the specification as probe 1618
PROPERTIES: Probe for selective detection of Borrelia burgdorferi
SEQUENCE:

CCTCATTTAT AAAAGAATTT TACAATCTTT CGACC

### SEQUENCE LISTING

SEQ. ID NO: 4
SEQUENCE LENGTH: 29 bases
SEQUENCE TYPE: Nucleotide
STRANDEDNESS: single
TOPOLOGY: Linear
MOLECULAR TYPE: synthetic or biological DNA or RNA
HYPOTHETICAL: No
SENSE OR ANTI-SENSE: Sense
FEATURES: Referred to in the specification as probe 1619
PROPERTIES: Probe for selective detection of Borrelia burgdorferi
SEQUENCE:

CATCACTTTG TCATTTCCTA CAAAGCTTA

### SEQUENCE LISTING

SEQ. ID NO: 5
SEQUENCE LENGTH: 32 bases
SEQUENCE TYPE: Nucleotide
STRANDEDNESS: single
TOPOLOGY: Linear
MOLECULAR TYPE: synthetic or biological DNA or RNA
HYPOTHETICAL: No
SENSE OR ANTI-SENSE: Sense
FEATURES: Referred to in the specification as probe 1620
PROPERTIES: Probe for selective detection of Borrelia burgdorferi
SEQUENCE:

GTTAGCTTCG GTACTAACTT TTAGTTAACA CC

### SEQUENCE LISTING

SEQ. ID NO: 6
SEQUENCE LENGTH: 36 bases
SEQUENCE TYPE: Nucleotide
STRANDEDNESS: single
TOPOLOGY: Linear
MOLECULAR TYPE: synthetic or biological DNA or RNA
HYPOTHETICAL: No
SENSE OR ANTI-SENSE: Sense
FEATURES: Referred to in the specification as probe 1621
PROPERTIES: Probe for selective detection of Borrelia burgdorferi
SEQUENCE:

CTTATCTGAG TCCCCACCAT TACATGCTGG TAACAG

### SEQUENCE LISTING

SEQ. ID NO: 7
SEQUENCE LENGTH: 64 bases
SEQUENCE TYPE: Nucleotide
STRANDEDNESS: single
TOPOLOGY: Linear
MOLECULAR TYPE: synthetic or biological DNA or RNA
HYPOTHETICAL: No
SENSE OR ANTI-SENSE: Sense
FEATURES: Referred to in the specification as probe 1622
PROPERTIES: Probe for selective detection of Borrelia burgdorferi
SEQUENCE:

CATACCTTAA ATACCTTCCT CCCTTACGGG TTAGAATAAT
AGCTTCGGGT ATCCTCAACT CGGG

### SEQUENCE LISTING

SEQ. ID NO: 8
SEQUENCE LENGTH: 30 bases
SEQUENCE TYPE: Nucleotide
STRANDEDNESS: single
TOPOLOGY: Linear
MOLECULAR TYPE: synthetic or biological RNA
HYPOTHETICAL: No
SENSE OR ANTI-SENSE: Sense
FEATURES: Target for probe 1616
ORIGINAL SOURCE ORGANISM: Borrelia burgdorferi
PROPERTIES: Region 63-106 of 16S rRNA of Borrelia burgdorferi
SEQUENCE:

CAAACGGGAU GUAGCAAUAC AUUCAGUGGC

### SEQUENCE LISTING

SEQ. ID NO: 9
SEQUENCE LENGTH: 29 bases
SEQUENCE TYPE: Nucleotide
STRANDEDNESS: single
TOPOLOGY: Linear
MOLECULAR TYPE: synthetic or biological RNA
HYPOTHETICAL: No
SENSE OR ANTI-SENSE: Sense
FEATURES: Target for probe 1617
ORIGINAL SOURCE ORGANISM: Borrelia burgdorferi
PROPERTIES: Region 178-194 of 16S rRNA of Borrelia burgdorferi
SEQUENCE:

AAUAAGGUCA GUUAAUUUGU UAAUUGAUG

### SEQUENCE LISTING

SEQ. ID NO: 10
SEQUENCE LENGTH: 35 bases
SEQUENCE TYPE: Nucleotide
STRANDEDNESS: single
TOPOLOGY: Linear
MOLECULAR TYPE: synthetic or biological RNA
HYPOTHETICAL: No
SENSE OR ANTI-SENSE: Sense
FEATURES: Target for probe 1618
ORIGINAL SOURCE ORGANISM: Borrelia burgdorferi
PROPERTIES: Region 416-450 of 16S rRNA of Borrelia burgdorferi
SEQUENCE:

GGUCGAAAGA UUGUAAAAUU CUUUUAUAAA UGAGG

### SEQUENCE LISTING

SEQ. ID NO: 11
SEQUENCE LENGTH: 29 bases
SEQUENCE TYPE: Nucleotide
STRANDEDNESS: single
TOPOLOGY: Linear
MOLECULAR TYPE: synthetic or biological RNA
HYPOTHETICAL: No
SENSE OR ANTI-SENSE: Sense
FEATURES: Target for probe 1619
ORIGINAL SOURCE ORGANISM: Borrelia burgdorferi
PROPERTIES: Region 453-481 of 16S rRNA of Borrelia burgdorferi
SEQUENCE:

UAAGCUUUGU AGGAAAUGAC AAAGUGUAG

### SEQUENCE LISTING

SEQ. ID NO: 12
SEQUENCE LENGTH: 32 bases
SEQUENCE TYPE: Nucleotide
STRANDEDNESS: single
TOPOLOGY: Linear
MOLECULAR TYPE: synthetic or biological RNA
HYPOTHETICAL: No
SENSE OR ANTI-SENSE: Sense
FEATURES: Target for probe 1620
ORIGINAL SOURCE ORGANISM: Borrelia burgdorferi
PROPERTIES: Region 829-866 of 16S rRNA of Borrelia burgdorferi
SEQUENCE:

GGUGUUAACU AAAAGUUAGU ACCGAAGCUA AC

### SEQUENCE LISTING

SEQ. ID NO: 13
SEQUENCE LENGTH: 36 bases
SEQUENCE TYPE: Nucleotide
STRANDEDNESS: single
TOPOLOGY: Linear
MOLECULAR TYPE: synthetic or biological RNA
HYPOTHETICAL: No
SENSE OR ANTI-SENSE: Sense
FEATURES: Target for probe 1621
ORIGINAL SOURCE ORGANISM: Borrelia burgdorferi
PROPERTIES: Region 1122-1156 of 16S rRNA of Borrelia burgdorferi
SEQUENCE:

CUGUUACCAG CAUGUAAUGG UGGGGACUCA GAUAAG

### SEQUENCE LISTING

SEQ. ID NO: 14
SEQUENCE LENGTH: 61 bases
SEQUENCE TYPE: Nucleotide
STRANDEDNESS: single
TOPOLOGY: Linear
MOLECULAR TYPE: synthetic or biological RNA
HYPOTHETICAL: No
SENSE OR ANTI-SENSE: Sense
FEATURES: Target for probe 1622
ORIGINAL SOURCE ORGANISM: Borrelia burgdorferi
PROPERTIES: Region 1414-1475 of 16S rRNA of Borrelia burgdorferi
SEQUENCE:

CCCGAGUUGA GGAUACCCGA AGCUAUUAUU CUAACCCGUA
AGGGAGGAAG GUAUUUAAGG U

### SEQUENCE LISTING

SEQ. ID NO: 15
SEQUENCE LENGTH: 37 bases
SEQUENCE TYPE: Nucleotide
STRANDEDNESS: single
TOPOLOGY: Linear
MOLECULAR TYPE: synthetic or biological DNA or RNA
HYPOTHETICAL: No
SENSE OR ANTI-SENSE: Sense
FEATURES: Features referred to in the specification as Probe/Primer 1643
ORIGINAL SOURCE ORGANISM: Borrelia burgdorferi
PROPERTIES: Probe/Primer for detection of Borrelia burgdorferi
SEQUENCE:

CCGAATTCGT CGACAACAGA GTTTGATCCT GGCTTAG

### SEQUENCE LISTING

SEQ. ID NO: 16
SEQUENCE LENGTH: 33 bases
SEQUENCE TYPE: Nucleotide
STRANDEDNESS: single
TOPOLOGY: Linear
MOLECULAR TYPE: synthetic or biological DNA or RNA
HYPOTHETICAL: No
SENSE OR ANTI-SENSE: Sense
FEATURES: Features referred to in the specification as Probe/Primer 1637
ORIGINAL SOURCE ORGANISM: Borrelia burgdorferi
PROPERTIES: Probe/Primer for detection of Borrelia burgdorferi
SEQUENCE:

CCCGGGATCC AAGCTTAAGG AGGTGATCCA GCC

### SEQUENCE LISTING

SEQ. ID NO: 17
SEQUENCE LENGTH: 20 bases
SEQUENCE TYPE: Nucleotide
STRANDEDNESS: single
TOPOLOGY: Linear
MOLECULAR TYPE: synthetic or biological DNA
HYPOTHETICAL: No
SENSE OR ANTI-SENSE: Sense
FEATURES: Target for Probe/Primer 1643
ORIGINAL SOURCE ORGANISM: Borrelia burgdorferi
PROPERTIES: 16S rDNA strand complementary to Borrelia 16S rRNA at its 5′ end
SEQUENCE:

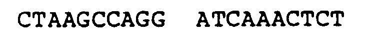

### SEQUENCE LISTING

SEQ. ID NO: 18
SEQUENCE LENGTH: 17 bases
SEQUENCE TYPE: Nucleotide
STRANDEDNESS: single
TOPOLOGY: Linear
MOLECULAR TYPE: synthetic or biological DNA
HYPOTHETICAL: No
SENSE OR ANTI-SENSE: Sense
FEATURES: Target for Probe/Primer 1637
ORIGINAL SOURCE ORGANISM: Borrelia burgdorferi
PROPERTIES: 16S rDNA strand complementary to Borrelia 16S rRNA at its 3′ end
SEQUENCE:

GGCTGGATCA CCTCCTT

## Claims

1. A nucleic acid fragment capable of hybridizing, under predetermined stringency conditions, to rRNA or rDNA genes (rDNA) of Borrelia burgdorferi wherein the fragment is complementary to at least 90% of a sequence comprising any ten consecutive nucleotides within a probe having one of the following nucleotide sequences:

Probe 1616:    5'-GCCACTGAATGTATTGCTACATCCCGTTG-3'

Probe 1617:    5'-CATCAATTAACAAATTAACTGACCTTATT-3'

Probe 1618:    5'-CCTCATTTATAAAAGAATTTTACAATCTTTCGACC-3'

Probe 1619:    5'-CATCACTTTGTCATTTCCTACAAAGCTTA-3'

Probe 1620:    5'-GTTAGCTTCGGTACTAACTTTTAGTTAACACC-3'

Probe 1621:    5'-CTTATCTGAGTCCCCACCATTACATGCTGGTAACAG-3'

Probe 1622:    5'-CATACCTTAAATACCTTCCTCCCTTACGGGTTAGAA
               TAATAGCTTCGGGTATCCTCAACTCGGG-3'

2. A nucleic acid fragment capable of hybridizing, under predetermined stringency conditions, to rRNA or rDNA genes (rDNA) of Borrelia burgdorferi wherein the fragment is homologous to at least 90% of a sequence comprising any ten consecutive nucleotides within Probe 1616, Probe 1617, Probe 1618, Probe 1619, Probe 1620, Probe 1621 or Probe 1622, the said probes being as defined in claim 1.

3. A nucleic acid sequence as claimed in claim 1 or claim 2 which comprises Probe 1616, Probe 1617, Probe 1618, Probe 1619, Probe 1620, Probe 1621 or Probe 1622 or one of their complementary sequences.

4. A set of probes comprising at least two nucleic acid fragments, as claimed in any one of claims 1 to 3.

5. A set of probes comprising a first nucleic acid that is a nucleic acid fragment as claimed in any one of claims 1 to 3, and a second nucleic acid that is probe/primer 1643 or probe/primer 1637, having the following nucleotide sequences

Probe/primer 1643: 5'-CCGAATTCGTCGACAACAGAGTTTGATCCTGG
CTTAG-3'

Probe/primer 1637: 5'-CCCGGATCCAAGCTTAAGGAGGTGATCC
AGCC-3'

6. A method for detecting the etiological agent of Lyme disease or borreliosis in a sample, comprising the steps of:
(a) contacting the sample with at least one nucleic acid fragment as claimed in any one of claims 1 to 3; under conditions that allow the fragment to hybridize to rRNA or rDNA of a Borrelia species causing Lyme disease, if present in the sample, whereby hybrid nucleic acid complexes are formed; and
(b) detecting the hybrid nucleic acid complexes as an indication of the presence of a Borrelia species.

7. A method for detecting the etiological agent of Lyme disease or borreliosis in a sample, comprising the steps
(a) contacting the sample with a nucleic acid fragment comprising probe/primer 1637 or probe/primer 1643, the said probe/primers being as defined in claim 5; under conditions that allow the fragment to hybridize to rRNA or rDNA of a Borrelia species causing Lyme disease, if present in the sample, whereby hybrid nucleic acid complexes are formed;
(b) detecting the hybrid nucleic acid complexes by additionally contacting the sample with a second nucleic acid fragment comprising Probe 1616, Probe 1617, Probe 1618, Probe 1619, Probe 1620, Probe 1621 or Probe 1622 or one of their complementary sequences; and
(c) amplifying the 16S rRNA gene sequence of the Borrelia species, if present, by polymerase chain reaction.

## Patentansprüche

1. Nucleinsäurefragment, das unter vorherbestimmten Stringenzbedingungen mit rRNA oder rDNA-Genen (rDNA) von Borrelia burgdorferi hybridisieren kann, wobei das Fragment zu mindestens 90 % einer Sequenz komplementär ist, umfassend 10 beliebige aufeinanderfolgende Nucleotide innerhalb einer Sonde mit einer der folgenden Nucleotidsequenzen:

Sonde 1616:    5'-GCCACTGAATGTATTGCTACATCCCGTTG-3'

Sonde 1617:    5'-CATCAATTAACAAATTAACTGACCTTATT-3'

Sonde 1618:    5'-CCTCATTTATAAAAGAATTTTACAATCTTTCGACC-3'

Sonde 1619:    5'-CATCACTTTGTCATTTCCTACAAAGCTTA-3'

Sonde 1620:    5'-GTTAGCTTCGGTACTAACTTTTAGTTAACACC-3'

Sonde 1621:    5'-CTTATCTGAGTCCCCACCATTACATGCTGGTAACAG-3'

Sonde 1622:    5'-CATACCTTAAATACCTTCCTCCCTTACGGTTAGAA
               TAATAGCTTCGGGTATCCTCAACTCGGG-3'

2. Nucleinsäurefragment, das unter vorherbestimmten Stringenzbedingungen mit rRNA oder rDNA-Genen (rDNA) von Borrelia burgdorferi hybridisieren kann, wobei das Fragment zu mindestens 90 % einer Sequenz homolog ist, umfassend 10 beliebige aufeinanderfolgende Nucleotide innerhalb der Sonden 1616, 1617, 1618, 1619, 1620, 1621 oder 1622, wobei die Sonden wie in Anspruch 1 definiert sind.

3. Nucleinsäuresequenz nach Anspruch 1 oder 2, umfassend die Sonden 1616, 1617, 1618, 1619, 1620, 1621 oder 1622 oder eine ihrer komplementären Sequenzen.

4. Sondensatz, umfassend mindestens zwei Nucleinsäurefragmente nach einem der Ansprüche 1 bis 3.

5. Sondensatz, umfassend eine erste Nucleinsäure, die ein Nucleinsäurefragment nach einem der Ansprüche 1 bis 3 ist, und eine zweite Nucleinsäure, die Sonde/Primer 1643 oder Sonde/Primer 1637 mit den folgenden Nucleotidsequenzen ist

Sonde/Primer 1643:  5'-CCGAATTCGTCGACAACAGAGTTTGATCCTGG
                    CTTAG-3'

Sonde/Primer 1637:  5'-CCCGGATCCAAGCTTAAGGAGGTGATCC
                    AGCC-3'

6. Verfahren zum Nachweis des ätiologischen Wirkstoffes der Lyme-Krankheit oder Borreliose in einer Probe, das die Schritte umfaßt:
(a) Inkontaktbringen der Probe mit mindestens einem Nucleinsäurefragment nach einem der Ansprüche 1 bis 3 unter Bedingungen, die die Hybridisierung des Fragments mit rRNA oder rDNA von einer die Lyme-Krankheit verursachenden Borrelia-Art erlauben, sofern diese in der Probe anwesend ist, wobei Hybrid-Nucleinsäurekomplexe gebildet werden; und
(b) Nachweis der Hybrid-Nucleinsäurekomplexe als Anzeichen für die Gegenwart einer Borrelia-Art.

7. Verfahren zum Nachweis des ätiologischen Wirkstoffes der Lyme-Krankheit oder Borreliose in einer Probe, das die Schritte umfaßt:
(a) Inkontaktbringen der Probe mit einem Nucleinsäurefragment, das die Sonde/den Primer 1637 oder die Sonde/den Primer 1643 umfaßt, wobei die Sonde/die Primer wie in Anspruch 5 definiert sind, unter Bedingungen, die die Hybridisierung des Fragments mit rRNA oder rDNA von einer die Lyme-Krankheit verursachenden Borrelia-Art erlauben, sofern diese in der Probe anwesend ist, wobei Hybrid-Nucleinsäurekomplexe gebildet werden;
(b) Nachweis der Hybrid-Nucleinsäurekomplexe durch zusätzliches Inkontaktbringen der Probe mit einem die Sonden 1616, 1617, 1618, 1619, 1620, 1621 oder 1622 oder eine ihrer komplementären Sequenzen umfassenden zweiten Nucleinsäurefragment; und
(c) Amplifikation der 16S rRNA-Gensequenz der Borrelia-Art, sofern diese anwesend ist, durch die Polymerasekettenreaktion.

## Revendications

1. Fragment d'acide nucléique capable de s'hybrider, dans des conditions de stringence prédéterminées, l'ARNr ou à des gènes d'ADNr (ADNr) de Borrelia burgdorferi, lequel fragment est complémentaire d'au moins 90% d'une séquence comprenant toute série de dix nucléotides consécutifs à l'intérieur d'une sonde ayant l'une des séquences de nucléotides suivantes :

Sonde 1616 : 5'-GCCACTGAATGTATTGCTACATCCCGTTG-3'

Sonde 1617 : 5'-CATCAATTAACAAATTAACTGACCTTATT-3'

Sonde 1618 : 5'-CCTCATTTATAAAAGAATTTTACAATCTTTCGACC-3'

Sonde 1619 : 5'-CATCACTTTGTCATTTCCTACAAAGCTTA-3'

Sonde 1620 : 5'-GTTAGCTTCGGTACTAACTTTTAGTTAACACC-3'

Sonde 1621 : 5'-CTTATCTGAGTCCCCACCATTACATGCTGGTAACAG-3'

Sonde 1622 : 5'-CATACCTTAAATACCTTCCTCCCTTACGGGTTAGAA
TAATAGCTTCGGGTATCCTCAACTCGGG-3'

2. Fragment d'acide nucléique capable de s'hybrider, dans des conditions de stringence prédéterminées, à l'ARNr ou à des gènes d'ADNr (ADNr) de Borrelia buradorferi, lequel fragment est homologue à au moins 90% d'une séquence comprenant toute série de dix nucléotides consécutifs à l'intérieur de la Sonde 1616, de la Sonde 1617, de la Sonde 1618, de la Sonde 1619, de la Sonde 1620, de la Sonde 1621 ou de la Sonde 1622, lesdites sondes étant telles que définies à la revendication 1.

3. Séquence d'acide nucléique selon la revendication 1 ou la revendication 2, qui comprend la Sonde 1616, la Sonde 1617, la Sonde 1618, la Sonde 1619, la Sonde 1620, la Sonde 1621 ou la Sonde 1622 ou l'une de leurs séquences complémentaires.

4. Jeu de sondes comprenant au moins deux fragments d'acide nucléique tels que définis à l'une des revendications 1 à 3.

5. Jeu de sondes comprenant un premier acide nucléique qui est un fragment d'acide nucléique tel que défini à l'une des revendications 1 à 3, et un second acide nucléique qui est la sonde/amorce 1643 ou la sonde/amorce 1637, ayant les séquences nucléotidiques suivantes :

Sonde/amorce 1643 : 5'-CCGAATTCGTCGACAACAGAGTTTGATCCTGG
CTTAG-3'

Sonde/amorce 1637 : 5'-CCCGGATCCAAGCTTAAGGAGGTGATCC
AGCC-3'

6. Procédé pour détecter l'agent étiologique de la maladie de Lyme ou de la borréliose dans un échantillon, comprenant les étapes de :
(a) mise en contact de l'échantillon avec au moins un fragment d'acide nucléique tel que défini à l'une des revendications 1 à 3 ; dans des conditions qui permettent au fragment de s'hybrider à l'ARNr ou à l'ADNr d'une espèce Borrelia provoquant la maladie de Lyme, si elle est présente dans l'échantillon, ce par quoi des complexes hybrides d'acide nucléique sont formés ; et
(b) détection des complexes hybrides d'acide nucléique en tant qu'indication de la présence d'une espèce Borrelia.

7. Procédé pour détecter l'agent étiologique de la maladie de Lyme ou de la borréliose dans un échantillon, comprenant les étapes de :
(a) mise en contact de l'échantillon avec un fragment d'acide nucléique comprenant la sonde/amorce 1637 ou la sonde/amorce 1643, lesdites sondes/amorces étant telles que définies à la revendication 5, dans des conditions qui permettent au fragment de s'hybrider à l'ARNr ou à l'ADNr d'une espèce Borrelia provoquant la maladie de Lyme, si elle est présente dans l'échantillon, ce par quoi des complexes hybrides d'acide nucléique sont formés ;
(b) détection des complexes hybrides d'acide nucléique par mise en contact supplémentaire de l'échantillon avec un second fragment d'acide nucléique comprenant la Sonde 1616, la Sonde 1617, la Sonde 1618, la Sonde 1619, la Sonde 1620, la Sonde 1621 ou la Sonde 1622 ou l'une de leurs séquences complémentaires ; et
(c) l'amplification de la séquence génique d'ARNr 16S de l'espèce Borrelia, si elle est présente, par réaction en chaîne de polymérase.
